# EUROPEAN PATENT APPLICATION

(11) **EP 1 374 695 A1**
(43) Date of publication of application: **02.01.2004**
(21) Application number: 03253801.9
(22) Date of filing: 17.06.2003
(51) Int. Cl.: A23K 1/18, C12N 1/16, C12N 13/00, A61K 35/66, A23K 1/00

(54) **Feed additives for sheep**

(30) Priority: 18.06.2002 US 175053
(71) Applicant: Ultra Biotech Limited, Douglas IM1 1SA, Isle of Man (GB)
(72) Inventor: Cheung, Ling Yuk, New Territories, Hong Kong (CN)
(74) Representative: Baldock, Sharon Claire

(57) **Abstract**

The present invention relates to feed additives for sheep. The invention provides methods for making a biological compositions comprising yeast cells that can improve the immune functions of sheep. The invention also relates to methods for manufacturing the biological compositions, and methods of using the biological compositions as feed additives.

## Description

### 1. FIELD OF THE INVENTION

The invention relates to biological compositions comprising yeast cells that can improve the immune functions of animals. The invention also relates to methods for manufacturing the biological compositions, and methods of using the biological compositions as animal feed additives.

### 2. BACKGROUND OF THE INVENTION

Antibiotics have been added to animal feed since the 1940s. It has been reported in 2000 that more than a third of the antibiotics sold in the United States--about 18 million pounds a year- are used in animal husbandry. They are used to treat sick animals; to prevent other animals housed in confined barns or coops from infections; and to make the animals grow faster. In terms of volume, most antibiotics are used for the first two reasons. Only 6.1 percent of the drugs goes toward growth promotion. However, in terms of the number of animals exposed, the role of growth promotion is huge. That is because farmers give antibiotics, in low but daily doses, to entire herds or flocks. Regular low doses of antibiotics not only help keep livestock healthy, but also improve the absorption of nutrients, which helps the animals grow faster on less feed, and thus increase profits, particularly in intensive farming operations. It has been estimated that 75 percent of the 92 million pigs in the United States routinely intake feed laced with antibiotics. So do about 6 percent of cattle, 25 percent of chickens and half the turkeys.

It is known that excess antibiotics and chemicals including those that are not metabolized by the animals can either remain in the body or be excreted. In the first instance, it may accumulate in the meat (and milk in the case of cows or goats) which will be consumed by humans. Thus, there is a possibility that these antibiotics and chemicals will contaminate human food products. Secondly and more importantly, it exposes microorganisms to the antibiotics, allowing antibiotic-resistant strains of the microorganisms to develop. If excreted, these antibiotics and chemicals will be released to the environment where they can come into contact with soil microorganisms. It has been hypothesized that over a period of time, commonly-used antibiotics will be render less effective against a range of microorganisms because of the development of antibiotic resistance and the transfer of such resistance to microorganisms including those that cause infections in humans.

There is growing evidence to suggest that the antibiotics widely used on farm animals are diminishing the power of important antibiotics to treat human infectious diseases especially those caused by food-borne pathogens. Farm animals treated with low levels of antibiotics are developing drug-resistant forms of bacteria. In one instance, while Synercid was approved for human use only in 1990's, a closely related drug called virginiamycin has been used on livestock since 1974. In fact, since 1990 there have been dramatic increase in the occurrence of multiply drug-resistant strains of zoonotic pathogens causing infections in humans in many developed countries. Of particular note has been the epidemic spread of MR strains *of Salmonella typhimurium* DT 104, which now appear to have an almost world-wide distribution. Among the DT104 strains, the increasing spectrum of resistance is of considerable concern. In many parts of the world, there is an increased incidence of decreased susceptibility to ciprofloxacin. For *Campylobacters* species, the incidence of ciprofloxacin-resistant organisms is also increasing, with reports of such isolates from numerous countries throughout the world. See Threllfall E.J. et al., Acta Vet Scand Suppl 2000; 93:63-8; Wegener H.C., N Engl J Med. 1999 May 20; 340(20):1525-32; Smith K.E. et al., N Engl J Med. 1999 May 20; 340(20):1581-2; Wegener H.C. et al., Acta Vet Scand Suppl. 1999; 92:51-7.

Because of concerns over the development of drug-resistance in microorganisms that cause human diseases, regulatory authorities in the United States and the European Union has banned or proposed banning the use of certain antibiotics in animal feed as a growth promoter. In defense, farmers and pharmaceutical makers argued that reducing the use of antibiotics would lead to increased disease and mortality rates and make meat more expensive by increasing the amount of time it takes to get animals to ideal slaughtering weight. The antibiotics help animals to get fatter quicker because they do not waste energy fighting illness. It has been argued that it is a ban on growth promoters that could pose the greatest risk to health. Without their protection, animals could face more serious disease. As a result, veterinarians would have to resort to high doses of therapeutic antibiotics. For example, the year following prohibition of antibiotics use in Sweden, an extra 50,000 pigs died of a form of diarrhoea.

It is clear that while the use of antibiotics as prophylactics in animal husbandry is banned or reduced, an urgent need for alternative means to reduce the incidence of infectious diseases in farm animals is emerging. The present invention provides a solution that uses yeasts to improve the immune functions of animals.

The inclusion of fungal cells or fungal fermentation products in animal feed have been in use for some time. Certain bacteria, yeasts and mold preparations, commonly referred to as probiotics or direct fed microbials, are administered orally or added to animal feeds to provide various benefits. However, the mechanism of action of such preparations are not properly understood but it is believed that they act by altering the gut microflora/microbiota of the animals thereby improving the health of the intestinal tract lining and allowing for improved nutrient absorption. In the case of ruminants, the preparation may ameliorate fermentation in the rumen that results in the wasteful production of gases.

For examples of the use of fungal cells and products in animal feed, see the annual Feed Additive Compendium published by The Miller Publishing Company (Minnetonka, Minn.) or the following patent literature:
United States Patent No. 3,903,307 discloses a process for making animal feed that is based on the fermentation of waste molasses or bagasse by yeasts, such as *Candida utilis* and *Trichoderma veride.*
U.S. Patent No. 4,055,667 discloses a liquid animal feed supplement that comprises a colloidal mixture of spent brewer's yeast in an aqueous alcoholic medium.
U.S. Patent No. 4,582,708 discloses an animal feed supplement that comprises live yeast cells *(Saccharomyces* species) having fermenting activity, a texturizing component comprising ground meal, ground legumes or mixtures thereof, a mineral mixture, a liquid binder, a vitamin mixture, and ground montmorillonite.
U.S. Patent No. 5,624,686 discloses an animal feed additive that is prepared by cultivating certain bacteria or yeast species (such as *Saccharomyces cerevisiae, Candida utilis)* and disrupting the microbial cells such that metabolites of the cells are efficiently made available to the animal.
U.S. Patent No. 6,214,337 discloses an animal feed that comprises yeast glucan which is derived from the cell walls of various yeast species (such as *Saccharomyces cerevisiae, Candida utilis).*
Citation of documents herein is not intended as an admission that any of the documents cited herein is pertinent prior art, or an admission that the cited documents are considered material to the patentability of the claims of the present application. All statements as to the date or representations as to the contents of these documents are based on the information available to the applicant and does not constitute any admission as to the correctness of the dates or contents of these documents.

### 3. SUMMARY OF THE INVENTION

The present invention relates to biological compositions that can be added to animal feed to reduce the incidence of infectious diseases in small ruminants, such as sheep.

In one embodiment, the present invention provides biological compositions comprising a plurality of live yeast cells which are capable of improving the immune functions of small ruminants, such as sheep and goats, when ingested. The biological compositions can be used for reducing the incidence of infectious diseases in sheep or optimizing the health of sheep.

In another embodiment, the invention provides methods of making the biological composition. In particular, the methods of the invention comprise culturing yeast cells in the presence of a series of electromagnetic fields of defined frequencies and field strengths, such that the yeast cells becomes metabolically active and potent at stimulating an animal's immune system. Up to four different components of yeast cells can be used to form the biological compositions. The yeast cells can also be subjected to a conditioning step to improve its performance. The conditioning step comprises culturing the yeast cells in a culture medium comprising gastric juice of an animal, wild hawthorn juice, and wild jujube juice. Methods for manufacturing the biological compositions comprising culturing the yeast cells under activation conditions, mixing various yeast cell cultures of the present invention, followed by drying the yeast cells and packing the final product, are encompassed. In preferred embodiments, the starting yeast cells are commercially available and/or accessible to the public, such as but not limited to *Saccharomyces cerevisiae.*

The biological compositions of the invention can be fed directly to animals or used as an additive to be incorporated into regular animal feed. Animal feed compositions comprising activated yeast cells of the invention and ingredients such as zeolite powder are encompassed by the invention.

### 4. BRIEF DESCRIPTION OF FIGURES

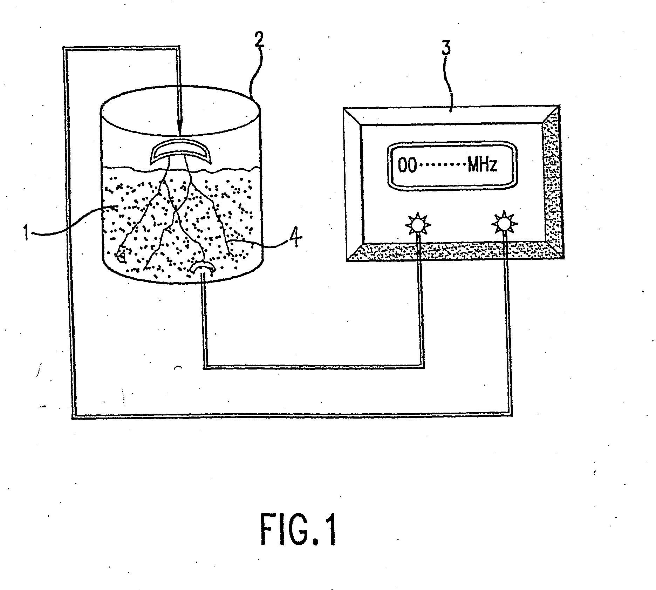

Fig. 1 Activation and conditioning of yeast cells. 1 yeast cell culture; 2 container; 3 electromagnetic field source; 4 electrode.

### 5. DETAILED DESCRIPTION OF THE INVENTION

The present invention relates to biological compositions that can improve the immune functions of animals, and/or reduce the incidence of infectious diseases. The present invention provides methods for manufacturing the biological compositions as well as methods for using the biological compositions as animal feed additives.

The biological compositions of the invention comprise yeasts. Unlike the traditional use of yeasts as a component of the feed, the yeast cells of the invention are not a primary source of nutrients for the animals. The yeast cells of the invention serve as a supplement to replace or reduce the antibiotics that are now routinely added to livestock feed. The yeast cells are live when administered orally or ingested along with feed by the animals. While in the gastrointestinal tract of an animal, the yeast cells are capable of stimulating the immune system and improving the immune functions of the animal, thereby reducing the incidence of infectious diseases. The use of the biological compositions of the invention can lower the overall cost of maintaining the health of animals in commercial animal operations, and make feasible the minimal use or the elimination of antibiotics in feed.

While the following terms are believed to have well-defined meanings in the art, the following are set forth to define the terms as used herein, and facilitate explanation of the invention.

As used herein, the term "feed" broadly refers to any kind of material, liquid or solid, that is used for nourishing an animal, and for sustaining normal or accelerated growth of an animal including newborns and young developing animals. Preferably, the feed is sheep feed which is generally suitable for small ruminants, such as sheep.

The term "animal" as used herein refers to small ruminants, such as sheep *(Ovis curies)* in general, but also includes but not limited to goats *(Capra hircus).*

The term "immune functions" as used herein broadly encompasses specific and non-specific immunological reactions of the animal, and includes both humoral and cell-mediated defense mechanisms. The immune functions of the animal enable the animal to survive and/or recover from an infection by a pathogen, such as bacteria, viruses, fungi, protozoa, helminths, and other parasites. The immune functions of the animal can also prevent infections, particularly future infections by the same pathogen after the animal had an initial exposure to the pathogen. Many types of immune cells are involved in providing the immune functions, which include various subsets of lymphocytes (B cells, T cells, K cells, NK cells), different types of leukocytes (macrophages, neutrophils, eosinophils, basophils), antigen presenting cells (dendritic cells, endothelial cells) and cells that are found in specialized organs and tissues with immunological activities (bone marrow, lymph nodes, thymus, bursa, Peyer's patch). Details of the immune system of ruminants are described in The Ruminant Immune System by John E. Butler, 1981, Perseus Books, which is incorporated herein by reference in its entirety.

In one embodiment, the present invention provides biological compositions that comprise at least one yeast cell component. Each yeast cell component comprises a population of live yeast cells which have been cultured under a specific set of conditions such that the yeast cells are capable of improving the immune functions of an animal. In preferred embodiments, the biological compositions of the invention comprise up to four yeast cell components.

According to the invention, under certain specific culture conditions, yeasts can be made metabolically active such that they become effective in stimulating and enhancing the immune functions of an animal which ingested the yeasts. Without being bound by any theory or mechanism, the inventor believes that the culture conditions activate and/or amplified the expression of a gene or a set of genes in the yeast cells such that the yeast cells becomes highly potent in stimulating the animal's immune system. It is envisioned that interactions between certain yeast gene products and elements of the animal's immune system is greatly enhanced by the elevated levels of these yeast gene products after the yeast cells have been cultured under the conditions described hereinbelow. These interactions are believed to involve immune cells lining the gastrointestinal tract, lymph nodes, as well as circulating immune cells. As a result of these interactions, the immune functions of the animals, such as responsiveness to and recovery from an infection, and resistance to diseases, are improved. The animals are protected from many types of infectious diseases, including parasitic diseases, such as but not limited to those caused by bacteria, viruses, fungi, protozoa, helminths, and the like. The benefits of using the biological compositions are demonstrated by experimental results obtained from animals which show resistance to or rapid recovery from an infectious disease.

In one embodiment, the biological compositions of the invention can be fed directly to an animal. In another embodiment, the biological compositions can be added to the feed. As known to those skilled in the relevant art, many methods and appliances may be used to mix the biological compositions of the invention with feed. In a particular embodiment, a mixture of culture broths of the yeasts of the present invention are added directly to the feed just prior to feeding the animal. Dried powders of the yeasts can also be added directly to the feed just prior to feeding the animal. In yet another embodiment of the present invention, the yeast cells are mixed with the raw constituents of the feed with which the yeast cells become physically incorporated. The biological compositions may be applied to and/or mixed with the feed by any mechanized means which may be automated.

The amount of biological composition used depends in part on the feeding regimen and the type of feed, and can be determined empirically. For example, the useful ratio of biological composition to animal feed ranges from 0.1% to 1% by dry weight, preferably, 0.3 to 0.8%, and most preferably at about 0.5%. Although not necessary, the biological compositions of the invention can also be used in conjunction or in rotation with other types of supplements, such as but not limited to vitamins, minerals, and vaccines.

Described below in Section 5.1 and 5.2 are four yeast cell components of the invention and methods of their preparation. Section 5.3 describes the manufacture of the biological compositions of the invention which comprises at least one of the four yeast cell components.

### 5.1. PREPARATION OF THE YEAST CELL CULTURES

The present invention provides yeast cells that are capable of improving the immune functions of an animal which ingested the yeast cells. Up to four different yeast cell components can be combined to make the biological compositions.

A yeast cell component of the biological composition is produced by culturing a plurality of yeast cells in an appropriate culture medium in the presence of an alternating electromagnetic field or multiple alternating electromagnetic fields in series over a period of time. The culturing process allows yeast spores to germinate, yeast cells to grow and divide, and can be performed as a batch process or a continuous process. As used herein, the terms "alternating electromagnetic field", "electromagnetic field" or "EM field" are synonymous. An electromagnetic field useful in the invention can be generated by various means well known in the art. A schematic illustration of exemplary setups are depicted respectively in Fig. 1. An electromagnetic field of a desired frequency and a desired field strength is generated by an electromagnetic wave source (3) which comprises one or more signal generators that are capable of generating electromagnetic waves, preferably sinusoidal waves, and preferably in the frequency range of 1500 MHz -15000 MHz- Such signal generators are well known in the art. Signal generators capable of generating signal with a narrower frequency range can also be used. If desirable, a signal amplifier can also be used to increase the output signal, and thus the strength of the EM field.

The electromagnetic field can be applied to the culture by a variety of means including placing the yeast cells in close proximity to a signal emitter connected to a source of electromagnetic waves. In one embodiment, the electromagnetic field is applied by signal emitters in the form of electrodes that are submerged in a culture of yeast cells (1). In a preferred embodiment, one of the electrodes is a metal plate which is placed on the bottom of a non-conducting container (2), and the other electrode comprises a plurality of wires or tubes so configured inside the container such that the energy of the electromagnetic field can be evenly distributed in the culture. For an upright culture vessel, the tips of the wires or tubes are placed within 3 to 30 cm from the bottom of the vessel (i.e, approximately 2 to 10% of the height of the vessel from the bottom). The number of electrode wires used depends on both the volume of the culture and the diameter of the wire. For example, for a culture having a volume of 10 liter or less, two or three electrode wires having a diameter of between 0.5 to 2.0 mm can be used. For a culture volume of 10 liter to 100 liter of culture, the electrode wires or tubes can have a diameter of 3.0 to 5.0 mm. For a culture volume of 100 liter to 1000 liter, the electrode wires or tubes can have a diameter of 6.0 to 15.0 mm. For a culture having a volume greater than 1000 liter, the electrode wires or tubes can have a diameter of between 20.0 to 25.0 mm.

In various embodiments, yeasts of the genera *of Saccharomyces, Candida, Crebrothecium, Geotrichum, Hansenula, Kloeckera, Lipomyces, Pichia, Rhodosporidium, Rhodotorula Torulopsis, Trichosporon,* and *Wickerhamia* can be used in the invention.

Non-limiting examples of yeast strains include *Saccharomyces cerevisiae* Hansen, ACCC2034, ACCC2035, ACCC2036, ACCC2037, ACCC2038, ACCC2039, ACCC2040, ACCC2041, ACCC2042, AS2.1, AS2.4, AS2.11, AS2.14, AS2.16, AS2.56, AS2.69, AS2.70, AS2.93, AS2.98, AS2.101, AS2.109, AS2.110, AS2.112, AS2.139, AS2.173, AS2.174, AS2.182, AS2.196, AS2.242, AS2.336, AS2.346, AS2.369, AS2.374, AS2.375, AS2.379, AS2.380, AS2.382, AS2.390, AS2.393, AS2.395, AS2.396, AS2.397, AS2.398, AS2.399, AS2.400, AS2.406, AS2.408, AS2.409, AS2.413, AS2.414, AS2.415, AS2.416, AS2.422, AS2.423, AS2.430, AS2.431, AS2.432, AS2.451, AS2.452, AS2.453, AS2.458, AS2.460, AS2.463, AS2.467, AS2.486, AS2.501, AS2.502, AS2.503, AS2.504, AS2.516, AS2.535, AS2.536, AS2.558, AS2.560, AS2.561, AS2.562, AS2.576, AS2.593, AS2/594, AS2.614, AS2.620, AS2.628, AS2.631, AS2.666, AS2.982, AS2.1190, AS2.1364, AS2.1396, IFFI 1001, IFFI 1002, IFFI 1005, IFFI 1006, IFFI 1008, IFFI 1009, IFFI 1010, IFFI 1012, IFFI 1021, IFFI 1027, IFFI 1037, IFFI 1042, IFFI 1043, IFFI 1045, IFFI 1048, IFFI 1049, IFFI 1050, IFFI 1052, IFFI 1059, IFFI 1060, IFFI 1063, IFFI 1202, IFFI 1203, IFFI 1206, IFFI 1209, IFFI 1210, IFFI 1211, IFFI 1212, IFFI 1213, IFFI 1215, IFFI 1220, IFFI 1221, IFFI 1224, IFFI 1247, IFFI 1248, IFFI 1251, IFFI 1270, IFFI 1277, IFFI 1287, IFFI 1289, IFFI 1290, IFFI 1291, IFFI 1292, IFFI 1293, IFFI 1297, IFFI 1300, IFFI 1301, IFFI 1302, IFFI 1307, IFFI 1308, IFFI 1309, IFFI 1310, IFFI 1311, IFFI 1331, IFFI 1335, IFFI 1336, IFFI 1337, IFFI 1338, IFFI 1339, IFFI 1340, IFFI 1345, IFFI 1348, IFFI 1396, IFFI 1397, IFFI 1399, IFFI 1411, IFFI 1413; *Saccharomyces cerevisiae* Hansen Var. ellipsoideus (Hansen) Dekker, ACCC2043, AS2.2, AS2.3, AS2.8, AS2.53, AS2.163, AS2.168, AS2.483, AS2.541, AS2.559, AS2.606, AS2.607, AS2.611, AS2.612; *Saccharomyces chevalieri* Guillermond, AS2.131, AS2.213; *Saccharomyces delbrueckii,* AS2.285; *Saccharomyces delbrueckii* Lindner var. mongolicus Lodder et van Rij, AS2.209, AS2.1157; *Saccharomyces exiguous* Hansen, AS2.349, AS2.1158; *Saccharomyces fermentati* (Saito) Lodder et van Rij, AS2.286, AS2.343; *Saccharomyces logos* van laer et Denamur ex Jorgensen, AS2.156, AS2.327, AS2.335; *Saccharomyces mellis* Lodder et Kreger Van Rij, AS2.195; *Saccharomyces microellipsoides* Osterwalder, AS2.699; Saccharomyces *oviformis* Osterwalder, AS2.100; *Saccharomyces rosei* (Guilliermond) Lodder et kreger van Rij, AS2.287; *Saccharomyces rouxii* Boutroux, AS2.178, AS2.180, AS2.370, AS2.371; *Saccharomyces sake* Yabe, ACCC2045; *Candida arborea,* AS2.566; *Candida Krusei* (Castellani) Berkhout, AS2.1045; *Candida lambica(Lindner* et Genoud) van.Uden et Buckley, AS2.1182; *Candida lipolytica* (Harrison) Diddens et Lodder, AS2.1207, AS2.1216, AS2.1220, AS2.1379, AS2.1398, AS2.1399, AS2.1400; *Candida parapsilosis* (Ashford) Langeron et Talice, AS2.590; *Candida parapsilosis* (Ashford) et Talice Var. intermedia Van Rij et Verona, AS2.491; *Candida pulcherriman* (Lindner) Windisch, AS2.492; *Candida rugousa* (Anderson) Diddens et Loddeer, AS2.511, AS2.1367, AS2.1369, AS2.1372, AS2.1373, AS2.1377, AS2.1378, AS2.1384; *Candida tropicalis* (Castellani) Berkout, ACCC2004, ACCC2005, ACCC2006, AS2.164, AS2.402, AS2.564, AS2.565, AS2.567, AS2.568, AS2.617, AS2.1387; *Candida utilis* Henneberg Lodder et Kreger Van Rij, AS2.120, AS2.281, AS2.1180; *Crebrothecium ashbyii* (Guillermond) Routein, AS2.481, AS2.482, AS2.1197; *Geotrichum candidum* Link, ACCC2016, AS2.361, AS2.498, AS2.616, AS2.1035, AS2.1062, AS2.1080, AS2.1132, AS2.1175, AS2.1183; *Hansenula anomala* (Hansen) H et P sydow, ACCC2018, AS2.294, AS2.295, AS2.296, AS2.297, AS2.298, AS2.299, AS2.300, AS2.302, AS2.338, AS2.339, AS2.340, AS2.341, AS2.470, AS2.592, AS2.641, AS2.642, AS2.635, AS2.782, AS2.794; *Hansenula arabitolgens* Fang, AS2.887; *Hansenula jadinii* Wickerham, ACCC2019; *Hansenula saturnus* (Klocker) H et P sydow, ACCC2020; *Hansenula schneggii* (Weber) Dekker, AS2.304; *Hansenula subpelliculosa* Bedford, AS2.738, AS2.740, AS2.760, AS2.761, AS2.770, AS2.783, AS2.790, AS2.798, AS2.866; *Kloeckera apiculata* (Reess emend. Klocker) Janke, ACCC2021, ACCC2022, ACCC2023, AS2.197, AS2.496, AS2.711, AS2.714; *Lipomyces starkeyi* Lodder et van Rij, ACCC2024, AS2.1390; *Pichia farinosa* (Lindner) Hansen, ACCC2025, ACCC2026, AS2.86, AS2.87, AS2.705, AS2.803; *Pichia membranaefaciens* Hansen, ACCC2027, AS2.89, AS2.661, AS2.1039; *Rhodosporidium toruloides* Banno, ACCC2028; *Rhodotorula glutinis* (Fresenius) Harrison, ACCC2029, AS2.280, ACCC2030, AS2.102, AS2.107, AS2.278, AS2.499, AS2.694, AS2.703, AS2.704, AS2.1146; *Rhodotorula minuta* (Saito) Harrison, AS2.277; *Rhodotorula rubar (Demme)* Lodder, ACCC2031, AS2.21, AS2.22, AS2.103, AS2.105, AS2.108, AS2.140, AS2.166, AS2.167, AS2.272, AS2.279, AS2.282; *Saccharomyces carlsbergensis* Hansen, AS2.113, ACCC2032, ACCC2033, AS2.312, AS2.116, AS2.118, AS2.121, AS2.132, AS2.162, AS2.189, AS2.200, AS2.216, AS2.265, AS2.377, AS2.417, AS2.420, AS2.440, AS2.441, AS2.443, AS2.444, AS2.459, AS2.595, AS2.605, AS2.638, AS2.742, AS2.745, AS2.748, AS2.1042; *Saccharomyces uvarum Beijer,* IFFI 1023, IFFI 1032, IFFI 1036, IFFI 1044, IFFI 1072, IFFI 1205, IFFI 1207; *Saccharomyces willianus* Saccardo, AS2.5, AS2.7, AS2.119, AS2.152, AS2.293, AS2.381, AS2.392, AS2.434, AS2.614, AS2.1189; *Saccharomyces sp.,* AS2.311; *Saccharomyces ludwigii* Hansen, ACCC2044, AS2.243, AS2.508; *Saccharomyces sinenses* Yue, AS2.1395; *Schizosaccharomyces octosporus* Beijerinck, ACCC 2046, AS2.1148; *Schizosaccharomyces pombe* Linder, ACCC2047, ACCC2048, AS2.248, AS2.249, AS2.255, AS2.257, AS2.259, AS2.260, AS2.274, AS2.994, AS2.1043, AS2.1149, AS2.1178, IFFI 1056; *Sporobolomyces roseus* Kluyver et van Niel, ACCC 2049, ACCC 2050, AS2.619, AS2.962, AS2.1036, ACCC2051, AS2.261, AS2.262; *Torulopsis candida* (Saito) Lodder, ACCC2052, AS2.270; *Torulopsis famta* (Harrison) Lodder et van Rij, ACCC2053, AS2.685; *Torulopsis globosa* (Olson et Hammer) Lodder et van Rij, ACCC2054, AS2.202; *Torulopsis inconspicua* Lodder et van Rij, AS2.75; *Trichosporon behrendii* Lodder et Kreger van Rij, ACCC2055, AS2.1193; *Trichosporon capitatum* Diddens et Lodder, ACCC2056, AS2.1385; *Trichosporon cutaneum*(de Beurm et al.)Ota, ACCC2057, AS2.25, AS2.570, AS2.571, AS2.1374; *Wickerhamia fluoresens* (Soneda) Soneda, ACCC2058, AS2.1388. Yeasts of the *Saccharomyces* genus are generally preferred. Among strains of *Saccharomyces cerevisiae, Saccharomyces cerevisiae* Hansen is a preferred strain.

Generally, yeast strains useful for the invention can be obtained from private or public laboratory cultures, or publically accessible culture deposits, such as the American Type Culture Collection, 10801 University Boulevard, Manassas, VA 20110-2209 and the China General Microbiological Culture Collection Center (CGMCC), China Committee for Culture Collection of Microorganisms, Institute of Microbiology, Chinese Academy of Sciences, Haidian, P.O. Box 2714, Beijing, 100080, China.

Although it is preferred, the preparation of the yeast cell components of the invention is not limited to starting with a pure strain of yeast. Each yeast cell component may be produced by culturing a mixture of yeast cells of different species or strains. The constituents of a yeast cell component can be determined by standard yeast identification techniques well known in the art.

In various embodiments of the invention, standard techniques for handling, transferring, and storing yeasts are used. Although it is not necessary, sterile conditions or clean environments are desirable when carrying out the manufacturing processes of the invention. Standard techniques for handling animal blood and immune cells, and for studying immune functions of an animal are also used. Details of such techniques are described in Advances in Laboratory Methods: General Haematology, 2000, Assendelft et al., (Ed.), Arnold, Edward (Publisher); Handbook of Vertebrate Immunology, 1998, Pastoret et al. (Ed.), Academic Press, and Current Protocols In Immunology, 1991, Coligan, et al. (Ed), John Wiley & Sons, Inc., which are both incorporated herein by reference in their entireties.

In one embodiment, the yeast cells of the first yeast cell component are cultured in the presence of at least one alternating electromagnetic (EM) field with a frequency in the range of 7730 MHz to 7750 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 7730, 7731, 7732, 7733, 7734, 7735, 7736, 7737, 7738, 7739, 7740, 7741, 7742, 7743, 7744, 7745, 7746, 7747, 7748, 7749, or 7750 MHz.

The field strength of the EM field(s) is in the range of 2.5 to 230 mV/cm. In a preferred embodiment, the EM field(s) at the beginning of a series have a lower EM field strength than later EM field(s), such that the yeast cell culture are exposed to EM fields of progressively increasing field strength. Accordingly, the yeast cells can be cultured at the lower EM field strength (e.g., 40 to 70 mV/cm) for 20 to 72 hours and then cultured at the higher EM field strength (e.g., 75-230 mV/cm) for another 20 to 72 hours. The yeast culture can remain in the same container and use the same set of electromagnetic wave generator and emitters when switching from one EM field to another EM field.

The culture process can be initiated by inoculating 100ml of medium with 1ml of an inoculum of the selected yeast strain(s) at a cell density of about 10⁵ cells/ml. The starting culture is kept at 35°C to 37°C for 24 to 48 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 1 provides an exemplary medium for culturing the first yeast cell component of the invention.

**Table 1**

| Medium Composition | Quantity |
|---|---|
| Sucrose or glucose | 20.0g |
| K₂HPO₄ | 0.25g |
| MgSO₄•7H₂O | 0.2g |
| NaCl | 0.22g |
| CaSO₄•2H₂O | 0.5g |
| CaCO₃ | 6.0g |
| Urea | 0.2 to 5.0g |
| Peptone | 15 g |
| Animal serum | 2-5ml |
| Autoclaved water | 1000ml |

In general, carbohydrates such as sugars, for example, sucrose, glucose, fructose, dextrose, maltose, xylose, and the like and starches, can be used either alone or in combination as sources of assimilable carbon in the culture medium. The exact quantity of the carbohydrate source or sources utilized in the medium depends in part upon the other ingredients of the medium but, in general, the amount of carbohydrate usually varies between about 0.1% and 5% by weight of the medium and preferably between about 0.5% and 2%, and most preferably about 0.8%. These carbon sources can be used individually, or several such carbon sources may be combined in the medium. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl, and CaSO₄.

The animal serum, which is a fraction of blood that comprises white blood cell, can be prepared from whole blood (100-500 ml) by standard methods known in the art, such as density gradient centrifugation. For example, bovine serum, or preferably sheep serum is used. Red blood cells are separated and discarded. The serum is added to the culture medium after the medium has been autoclaved and cooled to about 45°C.

It should be noted that the composition of the media provided in Table 1 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., one or more weeks). At the end of the culturing process, the yeast cells which constitute the first yeast cell component of the invention may be recovered from the culture by various methods known in the art, and stored at a temperature below about 0°C to 4°C. The recovered yeast cells may also be dried and stored in powder form.

A non-limiting example of making a first yeast cell component of the invention with *Saccharomyces cerevisiae* strain AS2.666 is provided in Section 6 hereinbelow.

In another embodiment, the yeast cells of the second yeast cell component are cultured in the presence of at least one alternating electromagnetic (EM) field with a frequency in the range of 6840 MHz to 6860 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 6840, 6841, 6842, 6843, 6844, 6845, 6846, 6847, 6848, 6849, 6850, 6851, 6852, 6853, 6854, 6855, 6856, 6857, 6858, 6859, or 6860 MHz.

The field strength of the EM field(s) is in the range of 6.5 to 250 mV/cm. In a preferred embodiment, the EM field(s) at the beginning of a series have a lower EM field strength than later EM field(s), such that the yeast cell culture are exposed to EM fields of progressively increasing field strength. Accordingly, the yeast cells can be cultured at the lower EM field strength (e.g., 70 -150 mV/cm) for 30 to 72 hours and then cultured at the higher EM field strength (e.g., 150-260 mV./cm) for another 20 to 72 hours. The yeast culture can remain in the same container and use the same set of electromagnetic wave generator and emitters when switching from one EM field to another EM field.

The culture process can be initiated by inoculating 100ml of medium with 1ml of an inoculum of the selected yeast strain(s) at a cell density of about 10⁵ cells/ml. The starting culture is kept at 35°C to 37°C for 24 to 48 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 2 provides an exemplary medium for culturing the second yeast cell component of the invention.

**Table 2**

| Medium Composition | Quantity |
|---|---|
| Sucrose or soluble starch | 20.0g |
| K₂HPO₄ | 0.25g |
| MgSO₄•7H₂O | 0.2g |
| NaCl | 0.22g |
| CaCO₃ | 0.5g |
| Urea | 2.0g |
| Peptone | 15 g |
| Animal serum | 2 - 5ml |
| Autoclaved water | 1000ml |

In general, carbohydrates such as sugars, for example, sucrose, glucose, fructose, dextrose, maltose, xylose, and the like and starches, can be used either alone or in combination as sources of assimilable carbon in the culture medium. The exact quantity of the carbohydrate source or sources utilized in the medium depends in part upon the other ingredients of the medium but, in general, the amount of carbohydrate usually varies between about 0.1% and 5% by weight of the medium and preferably between about 0.5% and 2%, and most preferably about 0.8%. These carbon sources can be used individually, or several such carbon sources may be combined in the medium. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl, and CaSO₄.

The animal serum, which is a fraction of blood that comprises white blood cell, can be prepared from whole blood (100-500 ml) by standard methods known in the art, such as density gradient centrifugation. For example, bovine serum, or preferably sheep serum is used. Red blood cells are separated and discarded. The serum is added to the culture medium after the medium has been autoclaved and cooled to about 45 °C.

It should be noted that the composition of the media provided in Table 2 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., one or more weeks). At the end of the culturing process, the yeast cells which constitute the first yeast cell component of the invention may be recovered from the culture by various methods known in the art, and stored at a temperature below about 0°C to 4°C. The recovered yeast cells may also be dried and stored in powder form.

A non-limiting example of making a second yeast cell component of the invention with *Saccharomyces cerevisiae* strain AS2.614 is provided in Section 6 hereinbelow.

In yet another embodiment, the yeast cells of the third yeast cell component are cultured in the presence of at least one alternating electromagnetic (EM) field with a frequency in the range of 8228 MHz to 8250 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 8228, 8229, 8230, 8231, 8232, 8233, 8234, 8235, 8236, 8237, 8238, 8239, 8240, 8241, 8242, 8243, 8244, 8245, 8246, 8247, 8248, 8249 or 8250 MHz.

The field strength of the EM field(s) is in the range of 9.5 to 270 mV/cm. In a preferred embodiment, the EM field(s) at the beginning of a series have a lower EM field strength than later EM field(s), such that the yeast cell culture are exposed to EM fields of progressively increasing field strength. Accordingly, the yeast cells can be cultured at the lower EM field strength (e.g., 100-170 mV/cm) for 20 to 70 hours and then cultured at the higher EM field strength (e.g., 170-270 mV/cm) for another 20 to 70 hours. The yeast culture can remain in the same container and use the same set of electromagnetic wave generator and emitters when switching from one EM field to another EM field.

The culture process can be initiated by inoculating 100ml of medium with 1ml of an inoculum of the selected yeast strain(s) at a cell density of about 10⁵ cells/ml. The starting culture is kept at 35 °C to 37°C for 24 to 48 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 3 provides an exemplary medium for culturing the third yeast cell component of the invention.

**Table 3**

| Medium Composition | Quantity |
|---|---|
| Sucrose or soluble starch | 20.0g |
| MgSO₄•7H₂O | 0.25g |
| NaCl | 0.2g |
| Ca(H₂PO₄) | 0.22g |
| CaCO₃ | 0.5g |
| (NH₄)₂HPO₄ | 3.0g |
| K₂HPO₄ | 0.3g |
| Peptone | 15 g |
| Animal serum | 2 - 5ml |
| Autoclaved water | 1000ml |

In general, carbohydrates such as sugars, for example, sucrose, glucose, fructose, dextrose, maltose, xylose, and the like and starches, can be used either alone or in combination as sources of assimilable carbon in the culture medium. The exact quantity of the carbohydrate source or sources utilized in the medium depends in part upon the other ingredients of the medium but, in general, the amount of carbohydrate usually varies between about 0.1% and 5% by weight of the medium and preferably between about 0.5% and 2%, and most preferably about 0.8%. These carbon sources can be used individually, or several such carbon sources may be combined in the medium. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl, and CaSO₄.

The animal serum, which is a fraction of blood that comprises white blood cell, can be prepared from whole blood (100-500 ml) by standard methods known in the art, such as density gradient centrifugation. For example, bovine serum, or preferably sheep serum is used. Red blood cells are separated and discarded. The serum is added to the culture medium after the medium has been autoclaved and cooled to about 45 °C.

It should be noted that the composition of the media provided in Table 3 is not intended to be limiting. The process can be scaled up or down according to needs. Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence of the EM field(s) for an extended period of time (e.g., one or more weeks). At the end of the culturing process, the yeast cells which constitute the first yeast cell component of the invention may be recovered from the culture by various methods known in the art, and stored at a temperature below about 0°C to 4°C. The recovered yeast cells may also be dried and stored in powder form.

A non-limiting example of making a third yeast cell component of the invention with *Saccharomyces cerevisiae* strain AS2.504 is provided in Section 6 hereinbelow.

In yet another embodiment, the yeast cells of the fourth yeast cell component are cultured in the presence of at least one alternating electromagnetic (EM) field with a frequency in the range of 8410 MHz to 8430 MHz. A single EM field or a series of EM fields can be applied, each having a different frequency within the stated range, or a different field strength within the stated range, or different frequency and field strength within the stated ranges. Although any practical number of EM fields can be used within a series, it is preferred that, the yeast culture be exposed to a total of 2, 3, 4, 5, 6, 7, 8, 9 or 10 different EM fields in a series. The EM field(s), which can be applied by any means known in the art, can each have a frequency of 8410, 8411, 8412, 8413, 8414, 8415, 8416, 8417, 8418, 8419, 8420, 8421, 8422, 8423, 8424, 8425, 8426, 8427, 8428, 8429 or 8430 MHz.

The field strength of the EM field(s) is in the range of 22 to 340 mV/cm. In a preferred embodiment, the EM field(s) at the beginning of a series have a lower EM field strength than later EM field(s), such that the yeast cell culture are exposed to EM fields of progressively increasing field strength. Accordingly, the yeast cells can be cultured at the lower EM field strength (e.g., 160-250 mV/cm) for 20 to 56 hours and then cultured at the higher EM field strength (e.g., 260-340 mV/cm) for another 20 to 56 hours. The yeast culture can remain in the same container and use the same set of electromagnetic wave generator and emitters when switching from one EM field to another EM field.

The culture process can be initiated by inoculating 100ml of medium with 1ml of an inoculum of the selected yeast strain(s) at a cell density of about 10⁵ cells/ml. The starting culture is kept at 35°C to 37°C for 24 to 48 hours prior to exposure to the EM field(s). The culturing process may preferably be conducted under conditions in which the concentration of dissolved oxygen is between 0.025 to 0.08 mol/m³, preferably 0.04 mol/m³. The oxygen level can be controlled by any conventional means known in the art, including but not limited to stirring and/or bubbling.

The culture is most preferably carried out in a liquid medium which contains animal serum and sources of nutrients assimilable by the yeast cells. Table 4 provides an exemplary medium for culturing the fourth yeast cell component of the invention.

**Table 4**

| Medium Composition | Quantity |
|---|---|
| Starch | 20.0g |
| (NH₄)₂HPO₄ | 0.25g |
| K₂HPO₄ | 0.2g |
| MgSO₄•7H₂O | 0.22g |
| NaCl | 0.5g |
| CaSO₄•2H₂O | 0.3g |
| CaCO₃ | 3.0g |
| Peptone | 15g |
| Animal serum | 2 - 5ml |
| Autoclaved water | 1000ml |

In general, carbohydrates such as sugars, for example, sucrose, glucose, fructose, dextrose, maltose, xylose, and the like and starches, can be used either alone or in combination as sources of assimilable carbon in the culture medium. The exact quantity of the carbohydrate source or sources utilized in the medium depends in part upon the other ingredients of the medium but, in general, the amount of carbohydrate usually varies between about 0.1% and 5% by weight of the medium and preferably between about 0.5% and 2%, and most preferably about 0.8%. These carbon sources can be used individually, or several such carbon sources may be combined in the medium. Among the inorganic salts which can be incorporated in the culture media are the customary salts capable of yielding sodium, calcium, phosphate, sulfate, carbonate, and like ions. Non-limiting examples of nutrient inorganic salts are (NH₄)₂HPO₄, CaCO₃, MgSO₄, NaCl, and CaSO₄.

The animal serum, which is a fraction of blood that comprises white blood cell, can be prepared from whole blood (100-500 ml) by standard methods known in the art, such as density gradient centrifugation. For example, bovine serum, or preferably sheep serum is used. Red blood cells are separated and discarded. The serum is added to the culture medium after the medium has been autoclaved and cooled to about 45 °C.

It should be noted that the composition of the media provided in Table 4 is not intended to be limiting. The process can be scaled up or down according to needs. 5 Various modifications of the culture medium may be made by those skilled in the art, in view of practical and economic considerations, such as the scale of culture and local supply of media components.

Although the yeast cells will become activated even after a few hours of culturing in the presence of the EM field(s), the yeast cells can be cultured in the presence 0 of the EM field(s) for an extended period of time (e.g., one or more weeks). At the end of the culturing process, the yeast cells which constitute the first yeast cell component of the invention may be recovered from the culture by various methods known in the art, and stored at a temperature below about 0°C to 4°C. The recovered yeast cells may also be dried and stored in powder form.

5 A non-limiting example of making a fourth yeast cell component of the invention with *Saccharomyces cerevisiae* strain AS2.559 is provided in Seetion 6 hereinbelow.

### 5.2. CONDITIONING OF THE YEAST CELLS

In another aspect of the invention, performance of the activated yeast cells can be optimized by culturing the activated yeast cells in the presence of materials taken from the gastrointestinal tract of the type of animal to which the biological composition will be fed. The inclusion of this conditioning process allows the activated yeast cells to adapt to and endure the acidic environment of the animal's stomach.

According to the invention, activated yeast cells prepared as described in Section 5.1 can be further cultured in a medium with a composition as shown in Table 5.

**Table 5**

| (Per 1000 ml of culture medium) | |
|---|---|
| **Medium Composition** | **Quantity** |
| Gastric juice of sheep | 300ml; stored at 4°C |
| Wild jujube juice | 300ml |
| Wild hawthorn juice | 320ml |
| (NH₄)₂HPO₄ | 0.25g |
| K₂HPO₄ | 0.2g |
| MgSO₄•7H₂O | 0.22g |
| NaCl | 0.5g |
| CaSO₄•2H₂O | 0.3g |
| Peptone | 15 g |
| CaCO₃ | 3.0g |
| Yeast culture from first yeast cell component containing > 10⁸ cells/ml; see Table 1 | 20ml |
| Yeast culture from second yeast cell component containing > 10⁸ cells/ml; see Table 2 | 20ml |
| Yeast culture from third yeast cell component containing > 10⁸ cells/ml; see Table 3 | 20ml |
| Yeast culture from fourth yeast cell component containing > 10⁸ cells/ml; see Table 4 | 20ml |

The process can be scaled up or down according to needs.

The gastric juice of the animal, for example, a sheep, can be obtained from the liquid portion of the stomach content of a freshly slaughtered animal. The content of the stomach is filtered under sterile conditions to obtain a clear fluid which can be stored at 4°C before use.

The wild jujube juice is a filtered extract of wild jujube fruits prepared by mixing 5 ml of water per gram of crushed wild jujube. The wild hawthorn juice is a filtered extract of wild hawthorn fruits prepared by mixing 5 ml of water per gram of crushed wild hawthorn.

The mixture of yeast cells is cultured for about 48 to 96 hours in the presence of a series of electromagnetic fields. Each electromagnetic field has a frequency that, depending on the strains of yeast included, corresponds to one of the four ranges of frequencies described in Sections 5.1. If all four yeast components are present, a combination of the following four frequency bands can be used : 7730-7750 MHz; 6840-6860 MHz; 8228-8250 MHz; 8410-8430 MHz. The EM fields can be applied sequentially or simultaneously. Generally, the yeast cells are subjected to an EM field strength in the range from 85mV/cm to 320mV/cm in this process.

While the yeast cell culture is exposed to the EM field(s), the culture is incubated at temperatures that cycle between about 5°C to about 38°C. For example, in a typical cycle, the temperature of the culture may start at about 37°C and be allowed to fall gradually to about 5°C, and then gradually be brought up to about 38°C for another cycle. Each complete cycle lasts about 3 hours. At the end of the cycles, the activated and conditioned yeast cells can be recovered by centrifugation at about 3500 rpm and stored under 4°C.

### 5.3 MANUFACTURE OF THE BIOLOGICAL COMPOSITIONS

The present invention further provides a method for manufacturing a biological composition that comprises the yeast cells of the invention. Preferably, the biological compositions of the invention comprise yeast cells activated by the methods described in section 5.1 and which have been subject to conditioning by the method described in section 5.2. Most preferably, the biological compositions comprise all four yeast cell components.

To mass produce the biological compositions of the invention, the culture process is scaled up accordingly starting with activated yeast cell cultures as prepared in Section 5.1 and preferably conditioned as described in Section 5.2. To illustrate the scaled-up process, a method for producing 1000 kg of the biological composition is described as follows:

A stock culture of each of the four activated yeast cell components are added to a culture medium comprising 100 kg starch in 250 liters of water. The activated yeast cells are then cultured at 35 ° to 38°C in the presence of an EM field(s) of the respective frequencies and a field strength within 120 to 450 mV/cm. The culture process is carried out for about 48 to 96 hours, or when the yeast cell number reaches about 2 x 10¹⁰/ml. At this point, the activated yeast cells must be stored at about 15° to 20 °C, and if not used immediately, dried for storage within 24 hours. This process is repeated for each of the four yeast cell components. To make a biological composition comprising all four yeast cell components, 250 liters of culture media of each of the four activated yeast cell components (i.e, a total of 1000 liters) are mixed and combined with 600 kg of starch.

Since the yeast cells and the biological compositions are not necessarily used immediately, the prepared yeast cells and biological compositions can be dried in a two-stage drying process. During the first drying stage, the yeast cells are dried in a first dryer at a temperature not exceeding 65°C for a period of time not exceeding 10 minutes so that yeast cells quickly become dormant. The yeast cells are then sent to a second dryer and dried at a temperature not exceeding 70°C for a period of time not exceeding 30 minutes to further remove water. After the two stages, the water content should be lower than 5%. It is preferred that the temperatures and drying times be adhered to in both drying stages so that yeast cells do not lose their vitality and functions. The dried yeast cells are then cooled to room temperature. The dried yeast cells may also be screened in a separator so that particles of a preferred size are selected. The dried cells can then be sent to a bulk bag filler for packing.

### 6. EXAMPLE

The following example illustrates the manufacture of a biological composition that can be used as an animal feed additive.

The biological composition comprises the following four components of yeasts: *Saccharomyces cerevisiae* AS2.666, AS2.614, AS2.504 and AS2.559. Each component of yeast cells is capable of inhibiting the development of an infection by *Clostridium perifringens* and reducing the mortality of infected sheep. The four yeast cell components are prepared and tested separately as follows:

A starting culture containing about 10⁵ cells/ml of AS2.666 is placed into the container (2) as shown in Figure 1 containing a medium with the composition as shown in Table 1. Initially, the yeast cells are cultured for about 24 hours at 36 ± 1 °C without an EM field. Then, in the same medium, at 36 ± 1 °C, the yeast cells are cultured in the presence of a series of eight EM fields applied in the order stated: 7731MHz at 48mV/cm for 26 hrs; 7739MHz at 48mV/cm for 26 hrs; 7742MHz at 48mV/cm for 10 hrs; 7748MHz at 48mV/cm for 10 hrs; 7731MHz at 220mV/cm for 15 hrs; 7739MHz at 220mV/cm for 15 hrs; 7742MHz at 220mV/cm for 10 hrs; and 7748MHz at 220mV/cm for 10 hrs. The yeast cells were conditioned by further culturing in sheep gastric juice and hawthorn juice as described in section 5.2, in the presence of a series of two EM fields: 7731 MHz at 220 mV/cm for 15 hours and 7739 MHz at 220 mV/cm for 15 hours. After the last culture period, the yeast cells are either used within 24 hours to make the biological compositions, or dried for storage as described in section 5.3.

The beneficial effect of this first component of yeast cells on animals was tested as follows: The test was conducted with 360 lamb (Mongolian No. 3 breed), all about two months old, with body weight within ≤ 10%. The animals were divided into four groups each with 90 animals. The animals in all four groups were each injected with 5x10⁶ *Clostridium perifringens* type D that causes clostridiosis in the animals. The first group of animals (Group A) were fed a diet comprising a mixture of antibiotics as shown in Table 6.

**Table 6:**

| composition of animal feed containing antibiotics | | |
|---|---|---|
| Ingredients | Quantities per metric ton | Notes |
| Basic Feed (with no added antibiotics) | 1000 kg | as supplied by Beijing Dongfung Feed Factory |
| Dipterex (trichlorphone) | 300 g | |
| Salinomycin | 60 g | 600,000,000 units |
| Monensin | 80 g | 800,000,000 units |
| Neomycin | 150 g | 1,500,000,000 units |
| Chlprtetracycline | 100 g | 1,000,000,000 units |
| Oxytetracycline | 150 g | 1,500,000,000 units |
| Sulfamethazimun | 500 g | 500,000,000 units |
| Arsanilic acid | 100 g | |
| Praziquantel | 100 g | |

The animals of Group B were fed a diet comprising activated AS2.666 yeast cells. The activated yeast cells were present in an additive which was prepared by mixing dried cells with zeolite powder (less than 200 mesh) at a ratio of about 10⁹ yeast cells per gram of zeolite powder. For every 995 kg of basic feed, 5 kg of the feed additive was added, yielding an improved feed that comprises 0.5% additive by weight or 5 x 10¹² yeast cells. The third group of animals (Group C) was fed a diet which contains an additive that was prepared identically to that used in Group B except that the AS2.666 yeast cells were not activated. The animals of Group D were fed the basic diet with neither antibiotic nor yeast additives. After four months, the health status of the animals in various groups are shown in Table 7 below.

**Table 7:**

| Health status of animals fed with different diets | | | | | | |
|---|---|---|---|---|---|---|
| Group | Total/group | Total No. of sick animals | Very sick animals | Dead animals | Recovered animals | Not yet recovered |
| A | 90 | 9 | 6 | 6 | 0 | 3 |
| B | 90 | 10 | 7 | 4 | 5 | 1 |
| C | 90 | 62 | 33 | 29 | 3 | 30 |
| D | 90 | 63 | 35 | 33 | 1 | 29 |

To prepare the second component, a starting culture containing about 10⁵ cells/ml of AS2.614 is placed into the container (2) as shown in Figure 1 containing a medium with the composition as shown in Table 2. Initially, the yeast cells are cultured for about 22 hours at 36 ± 1°C without an EM field. Then, in the same medium, at 36 ± 1°C, the yeast cells are cultured in the presence of a series of eight EM fields applied in the order stated: 6841MHz at 87mV/cm for 18 hrs; 6847MHz at 87mV/cm for 18 hrs; 6853MHz at 87mV/cm for 18 hrs; 6858MHz at 87mV/cm for 18 hrs; 6841MHz at 245mV/cm for 10 hrs; 6847MHz at 245mV/cm for 10 hrs; 6853MHz at 245mV/cm for 10 hrs; and 6858MHz at 245mV/cm for 10 hrs. The yeast cells were conditioned by further culturing in sheep gastric juice/and hawthorn juice as described in section 5.2, in the presence of a series of two EM fields: 6841 MHz at 245 mV/cm for 10 hours and 6847 MHz at 245 mV/cm for 10 hours. After the last culture period, the yeast cells are either used within 24 hours to make the biological compositions, or dried for storage as described in section 5.3.

The beneficial effect of this second component of yeast cells on animals was tested as follows: The test was conducted with 360 lamb (Mongolian No. breed meat), all about two months old, with body weight within ≤ 10% . The animals were divided into four groups each with 90 animals. The first group of animals (Group A) were fed a diet comprising a mixture of antibiotics as shown in Table 8.

**Table 8:**

| composition of animal feed containing antibiotics | | |
|---|---|---|
| Ingredients | Quantities per metric ton | Notes |
| Basic Feed (with no added antibiotics) | 1000 kg | as supplied by Beijing Dongfung Feed Factory |
| Dipterex (trichlorphone) | 300 g | |
| Salinomycin | 60 g | 600,000,000 units |
| Monensin | 80 g | 800,000,000 units |
| Neomycin | 150 g | 1,500,000,000 units |
| Chlortetracycline | 100 g | 1,000,000,000 units |
| Oxytetracycline | 150 g | 1,500,000,000 units |
| Sulfamethazinum | 500 g | 500,000,000 units |
| Arsanilic acid | 100 g | |
| Praziquantel | 100 g | |

The animals of Group B were fed a diet comprising activated AS2.614 yeast cells. The activated yeast cells were present in an additive which was prepared by mixing dried cells with zeolite powder (less than 200 mesh) at a ratio of 1 x 10⁹ yeast cells per gram of zeolite powder. For every 995 kg of basic feed, 5 kg of the additive was added, yielding an improved feed that comprises 0.5% additive by weight. The third group of animals (Group C) was fed a diet which contains an additive that was prepared identically to that used in Group B except that the AS2.614 yeast cells were not activated. The animals of Group D were fed the basic diet with neither antibiotic nor yeast additives. After four months, the health status of the animals in various groups are shown in Table 9 below.

**Table 9:**

| Health status of animals fed with different diets | | | | | | |
|---|---|---|---|---|---|---|
| Group | Total/group | Total No. of sick animals | Very sick animals | Dead animals | Recovered animals | Not yet recovered |
| A | 90 | 10 | 8 | 8 | 0 | 2 |
| B | 90 | 9 | 5 | 3 | 5 | 1 |
| C | 90 | 59 | 29 | 29 | 4 | 26 |
| D | 90 | 63 | 36 | 35 | 0 | 28 |

For the third yeast cell component, a starting culture containing about 10⁵ cells/ml of AS2.504 is placed into the container (2) as shown in Figure 1 containing a medium with the composition as shown in Table 3. Initially, the yeast cells are cultured for about 33 hours at 36 ± 1 °C without an EM field. Then, in the same medium, at 36 ± 1°C, the yeast cells are cultured in the presence of a series of eight EM fields applied in the order stated: 8228MHz at 113mV/cm for 25 hrs; 8231MHz at 113mV/cm for 25 hrs; 8240MHz at 113mV/cm for 10 hrs; 8247MHz at 113mV/cm for 10 hrs; 8228MHz at 256mV/cm for 13 hrs; 8231MHz at 256mV/cm for 13 hrs; 8240MHz at 256mV/cm for 13 hrs; and 8247MHz at 256mV/cm for 13 hrs. The yeast cells were conditioned by further culturing in sheep gastric juice and hawthorn juice as described in section 5.2, in the presence of a series of two EM fields: 8228 MHz at 256 mV/cm for 13 hours and 8231 MHz at 256 mV/cm for 13 hours. After the last culture period, the yeast cells are either used within 24 hours to make the biological compositions, or dried for storage as described in section 5.3.

The beneficial effect of this third component of yeast cells on animals was ' tested as follows: The test was conducted with 360 lamb (Mongolian No. 3 breed), all about two months old, with body weight within ≤10%. The animals were divided into four groups each with 90 animals. The first group of animals (Group A) were fed a diet comprising a mixture of antibiotics as shown in Table 10.

**Table 10:**

| composition of animal feed containing antibiotics | | |
|---|---|---|
| Ingredients | Quantities per metric ton | Notes |
| Basic Feed (with no added antibiotics) | 1000 kg | as supplied by Beijing Dongfung Feed Factory |
| Dipterex (trichlorphone) | 300 g | |
| Salinomycin | 60 g | 600,000,000 units |
| Monensin | 80 g | 800,000,000 units |
| Neomycin | 150 g | 1,500,000,000 units |
| Chlortetracycline | 100 g | 1,000,000,000 units |
| Oxytetracycline | 150 g | 1,500,000,000 units |
| Sulfamethazinum | 500 g | 500,000,000 units |
| Arsanilic acid | 100 g | |
| Praziquantel | 100 g | |

The animals of Group B were fed a diet comprising activated AS2.504 yeast cells. The activated yeast cells were present in an additive which was prepared by mixing dried cells with zeolite powder (less than 200 mesh) at a ratio of 1 x 10⁹ yeast cells per gram of zeolite powder. For every 995 kg of basic feed, 5 kg of the additive was added, yielding an improved feed that comprises 0.5% additive by weight. The third group of animals (Group C) was fed a diet which contains an additive that was prepared identically to that used in Group B except that the AS2.504 yeast cells were not activated. The animals of Group D were fed the basic diet with neither antibiotic nor yeast additives. After four months, the health status of the animals in various groups are shown in Table 11 below.

**Table 11:**

| Health status of animals fed with different diets | | | | | | |
|---|---|---|---|---|---|---|
| Group | Total/group | Total No. of sick animals | Very sick animals | Dead animals | Recovered animals | Not yet recovered |
| A | 90 | 8 | 6 | 6 | 0 | 2 |
| B | 90 | 9 | 4 | 2 | 6 | 1 |
| C | 90 | 67 | 33 | 31 | 3 | 34 |
| D | 90 | 69 | 37 | 33 | 0 | 36 |

To prepare the fourth component, a starting culture containing about 10⁵ cells/ml of AS2.559 is placed into the container (2) as shown in Figure 1 containing a medium with the composition as shown in Table 4. Initially, the yeast cells are cultured for about 35 hours at 36 ± 1°C without an EM field. Then, in the same medium, at 36 ± 1°C, the yeast cells are cultured in the presence of a series of eight EM fields applied in the order stated: 8410MHz at 169mV/cm for 16 hrs; 8415MHz at 169mV/cm for 16 hrs; 8420MHz at 169mV/cm for 12 hrs; 8428MHz at 169mV/cm for 12 hrs; 8410MHz at 315mV/cm for 15 hrs; 8415MHz at 315mV/cm for 15 hrs; 8420MHz at 315mV/cm for 10 hrs; and 8428MHz at 315mV/cm for 10 hrs. The yeast cells were conditioned by further culturing in sheep gastric juice and hawthorn juice as described in section 5.2, in the presence of a series of two EM fields: 8410 MHz at 315 mV/cm for 15 hours and 8415 MHz at 315 mV/cm for 15 hours. After the last culture period, the yeast cells are either used within 24 hours to make the biological compositions, or dried for storage as described in section 5.3.

The beneficial effect of this fourth component of yeast cells on animals was tested as follows: The test was conducted with 360 lamb (Mongolian No. 3 breed), all about two months old, with body weight within ≤ 10%. The animals were divided into four groups each with 30 animals. The first group of animals (Group A) were fed a diet comprising a mixture of antibiotics as shown in Table 12.

**Table 12:**

| composition of animal feed containing antibiotics | | |
|---|---|---|
| Ingredients | Quantities per metric ton | Notes |
| Basic Feed (with no added antibiotics) | 1000 kg | as supplied by Beijing Dongfung Feed Factory |
| Dipterex (trichlorphone) | 300 g | |
| Salinomycin | 60 g | 600,000,000 units |
| Monensin | 80 g | 800,000,000 units |
| Neomycin | 150 g | 1,500,000,000 units |
| Chlortetracycline | 100 g | 1,000,000,000 units |
| Oxytetracycline | 150 g | 1,500,000,000 units |
| Sulfamethazinum | 500 g | 500,000,000 units |
| Arsanilic acid | 100 g | |
| Praziquantel | 100 g | |

The animals of Group B were fed a diet comprising activated AS2.559 yeast cells. The activated yeast cells were present in an additive which was prepared by mixing dried cells with zeolite powder (less than 200 mesh) at a ratio of 1 x 10⁹ yeast cells per gram of zeolite powder. For every 995 kg of basic feed, 5 kg of the additive was added, yielding an improved feed that comprises 0.5% additive by weight. The third group of animals (Group C) was fed a diet which contains an additive that was prepared identically to that used in Group B except that the AS2.559 yeast cells were not activated. The animals of Group D were fed the basic diet with neither antibiotic nor yeast additives. After four months, the health status of the animals in various groups are shown in Table 13 below.

**Table 13:**

| Health status of animals fed with different diets | | | | | | |
|---|---|---|---|---|---|---|
| Group | Total/group | Total No. of sick animals | Very sick animals | Dead animals | Recovered animals | Not yet recovered |
| A | 90 | 13 | 7 | 5 | 1 | 5 |
| B | 90 | 9 | 3 | 2 | 5 | 22 |
| C | 90 | 65 | 31 | 30 | 2 | 33 |
| D | 90 | 69 | 34 | 31 | 1 | 37 |

A biological feed additive comprising all four yeast cell components was prepared by mixing dried cells of each component with zeolite powder (less than 200 mesh) at a ratio of 1 x 10⁹ yeast cells per gram of zeolite powder. For every 995 kg of basic feed, 5 kg of the yeast and zeolite powder mixture was added, yielding an additive that comprises 0.5% yeast and zeolite powder by weight. The beneficial effect of this biological feed additive on animals was tested with 360 lamb (a Mongolian No. 3 breed), all about two months old, with body weight within ≤ 10%. The animals were divided into four groups each with 30 animals. The first group of animals (Group A) were fed a diet comprising a mixture of antibiotics as shown in Table 14.

**Table 14:**

| composition of animal feed containing antibiotics | | |
|---|---|---|
| Ingredients | Quantities per metric ton | Notes |
| Basic Feed (with no added antibiotics) | 1000 kg | as supplied by Beijing Dongfung Feed Factory |
| Dipterex (trichlorphone) | 300 g | |
| Salinomycin | 60 g | 600,000,000 units |
| Monensin | 80 g | 800,000,000 units |
| Neomycin | 150 g | 1,500,000,000 units |
| Chlortetracycline | 100 g | 1,000,000,000 units |
| Oxytetracycline | 150 g | 1,500,000,000 units |
| Sulfamethazinum | 500 g | 500,000,000 units |
| Arsanilic acid | 100 g | |
| Praziquantel | 100 g | |

The animals of Group B were fed a diet comprising the biological feed additive prepared as described above. The third group of animals (Group C) was fed a diet which contains an additive that was prepared identically to that used in Group B except that the yeast cells were not activated. The animals of Group D were fed the basic diet with neither antibiotics nor biological feed additives. After four months, the health status of the animals in various groups are shown in Table 15 below.

**Table 15:**

| Health status of animals fed with different diets | | | | | | |
|---|---|---|---|---|---|---|
| Group | Total/group | Total No. of sick animals | Very sick animals | Dead animals | Recovered animals | Not yet recovered |
| A | 90 | 11 | 8 | 4 | 2 | 5 |
| B | 90 | 2 | 0 | 0 | 2 | 0 |
| C | 90 | 66 | 32 | 29 | 3 | 34 |
| D | 90 | 69 | 36 | 33 | 3 | 33 |

The above results indicate that the biological composition of the invention is a valuable animal feed additive that can be used to maintain the health of the animal, and help the animal recover from an infection.

The present invention is not to be limited in scope by the specific embodiments described which are intended as single illustrations of individual aspects of the invention, and functionally equivalent methods and components are within the scope of the invention. Indeed various modifications of the invention, in addition to those shown and described herein will become apparent to those skilled in the art from the foregoing description and accompanying drawings. Such modifications are intended to fall within the scope of the appended claims.

## Claims

1. A biological composition comprising at least one of the following yeast cell components:
(a) a first yeast cell component comprising a plurality of yeast cells that are prepared by culturing the yeast cells in an electromagnetic field or a series of electromagnetic fields having a frequency in the range of 7730 to 7750 Mhz and a field strength of 2.5 to 230 mV/cm;
(b) a second yeast cell component comprising a plurality of yeast cells that are prepared by culturing the yeast cells in an electromagnetic field or a series of electromagnetic fields having a frequency in the range of 6840 to 6860 Mhz and a field strength of 6.5 to 250 mV/cm;
(c) a third yeast cell component comprising a plurality of yeast cells that are prepared by culturing the yeast cells in an electromagnetic field or a series of electromagnetic fields having a frequency in the range of 8228 to 8250 Mhz and a field strength of 9.5 to 270 mV/cm; and
(d) a fourth yeast cell component comprising a plurality of yeast cells that are prepared by culturing the yeast cells in an electromagnetic field or a series of electromagnetic fields having a frequency in the range of 8410 to 8430 Mhz and a field strength of 22 to 340 mV/cm.

2. The biological composition of claim 1 which comprises the yeast cell components of (a), (b), (c) and (d).

3. The biological composition of claim 1 or 2, wherein the yeast cells are cells of Saccharomyces.

4. The biological composition of any one of claims 1 to 3, wherein the yeast cells are cells of *Saccharomyces cerevisiae.*

5. The biological composition of any preceding claims in which the yeast cells are dried.

6. An animal feed composition comprising the biological composition of any preceding claim and sheep feed.

7. The animal feed composition of claim 6 wherein the biological composition further comprises zeolite powder at a ratio of about 10⁹ yeast cells to 1 g of zeolite powder.

8. The animal feed composition of claim 7 in which 0.5% by weight is the biological composition of claim 1 or 2.

9. A method of preparing a biological composition, said method comprising culturing a plurality of yeast cells in an electromagnetic field or a series of electromagnetic fields having a frequency in the range of 7730 to 7750 MHz and a field strength of 2.5 to 230 mV/cm.

10. A method for preparing a biological composition, said method comprising culturing a plurality of yeast cells in an electromagnetic field or a series of electromagnetic fields having a frequency in the range of 6840 to 6860 MHz and a field strength of 6.5 to 250 mV/cm.

11. A method for preparing a biological composition, said method comprising culturing a plurality of yeast cells in an electromagnetic field or a series of electromagnetic fields having a frequency in the range of 8228 to 8250 MHz and a field strength of 9.5 to 270 mV/cm.

12. A method for preparing a biological composition, said method comprising culturing a plurality of yeast cells in an electromagnetic field or a series of electromagnetic fields having a frequency in the range of 8410 to 8430 MHz and a field strength of 15 to 340 mV/cm.

13. The method of any one of claims 9, 10, 11 or 12 wherein said method further comprises culturing the plurality of yeast cells in one or more of the electromagnetic fields in a culture medium comprising gastric juice of sheep, wild hawthorn juice and wild jujube juice.

14. A method of making an animal feed composition, said method comprising
(a) preparing one or more of the yeast cell components of claim 1,
(b) drying the yeast cell components of (a), and
(c) mixing the dried yeast cells with zeolite powder and sheep feed.

15. The method of claim 14, wherein the drying step comprises (i) drying at a temperature not exceeding 65°C for a period of time such that the yeast cells become dormant; and (b) drying at a temperature not exceeding 70°C for a period of time to reduce the moisture content to below 5%.

16. A method for reducing the incidence of infectious diseases in a sheep comprising feeding the sheep for a period of time an animal feed composition comprising at least one of the following yeast cell components:
(a) a first year cell component comprising a plurality of yeast cells that are prepared by culturing the yeast cells in an electromagnetic field or a series of electromagnetic fields having a frequency in the range of 7730 to 7750 MHz and a field strength of 2.5 to 230 mV/cm;
(b) a second yeast cell component comprising a plurality of yeast cells that are prepared by culturing the yeast cells in an electromagnetic field or a series of electromagnetic fields having a frequency in the range of 6840 to 6860 MHz and a field strength of 6.5 to 250 mV/cm;
(c) a third yeast cell component comprising a plurality of yeast cells that are prepared by culturing the yeast cells in an electromagnetic field or a series of electromagnetic fields having a frequency in the range of 8228 to 8250 MHz and a field strength of 9.5 to 270 mV/cm; and
(d) a fourth yeast cell component comprising a plurality of yeast cells that are prepared by culturing the yeast cells in an electromagnetic field or a series of electromagnetic fields having a frequency in the range of 8410 to 8430 MHz and a field strength of 22 to 340 mV/cm.

17. The method of claim 16, wherein the animal feed composition comprises the yeast cell components of (a), (b), (c) and (d), and zeolite powder.

18. The method of claims 16 or 17, wherein said yeast cells are *Saccharomyces cerevisiae* cells.

19. The method of any one of claims 16 to 18, wherein the yeast cell components and zeolite powder comprises 0.5% by weight of the animal feed composition.

20. The composition of claim 1 or 2, wherein the plurality of yeast cells used in preparing the first yeast cell component comprise cells of *Saccharomyces cerevisiae* AS2.666, wherein the plurality of yeast cells used in preparing the second yeast cell component comprise cells of *Saccharomyces cerevisiae* AS2.614, wherein the plurality of yeast cells used in preparing the third yeast cell component comprise cells of *Saccharomyces cerevisiae* AS2.504, and wherein the plurality of yeast cells used in preparing the fourth yeast cell component comprise cells of *Saccharomyces* AS2.559.

21. The animal feed composition of claim 6, wherein the plurality of yeast cells used in preparing the first yeast cell component comprise cells of *Saccharomyces* cerevisiae AS2.666, wherein the plurality of yeast cells used in preparing the second yeast cell component comprise cells of *Saccharomyces cerevisiae* AS2.614, wherein the plurality of yeast cells used in preparing the third yeast cell component comprise cells of *Saccharomyces cerevisiae* AS2.504, and wherein the plurality of yeast cells used in preparing the fourth yeast cell component comprise cells of *Saccharomyces* AS2.559.

22. The use of at least one of the following yeast cell components:
(a) a first year cell component comprising a plurality of yeast cells that are prepared by culturing the yeast cells in an electromagnetic field or a series of electromagnetic fields having a frequency in the range of 7730 to 7750 MHz and a field strength of 2.5 to 230 mV/cm;
(b) a second yeast cell component comprising a plurality of yeast cells that are prepared by culturing the yeast cells in an electromagnetic field or a series of electromagnetic fields having a frequency in the range of 6840 to 6860 MHz and a field strength of 6.5 to 250 mV/cm;
(c) a third yeast cell component comprising a plurality of yeast cells that are prepared by culturing the yeast cells in an electromagnetic field or a series of electromagnetic fields having a frequency in the range of 8228 to 8250 MHz and a field strength of 9.5 to 270 mV/cm; and
(d) a fourth yeast cell component comprising a plurality of yeast cells that are prepared by culturing the yeast cells in an electromagnetic field or a series of electromagnetic fields having a frequency in the range of 8410 to 8430 MHz and a field strength of 22 to 340 mV/cm.
In the manufacture of an animal feed composition for reducing the incidence of infectious diseases in sheep.
